# EUROPEAN PATENT SPECIFICATION

(11) **EP 2 074 981 B1**
(45) Date of publication and mention of the grant of the patent: **16.05.2018**
(21) Application number: 07829525.0
(22) Date of filing: 03.10.2007
(51) Int. Cl.: A61H 7/00, A61H 9/00, A61B 5/0285, A61H 1/00, A63B 21/00, A61F 5/34

(54) **THERAPEUTIC DEVICE FOR INCREASING MUSCLE STRENGTH**
THERAPEUTISCHES GERÄT ZUR STÄRKUNG DER MUSKULATUR
APPAREIL THÉRAPEUTIQUE POUR AUGMENTER LA FORCE MUSCULAIRE

(30) Priority: 18.10.2006 JP 2006284428
(43) Date of publication of application: 01.07.2009
(73) Proprietor: Kaatsu Japan Co. Ltd., Tokyo 183-0016 (JP)
(72) Inventor: NAKAJIMA, Toshiaki, Tokyo 1138655 (JP); SATO, Yoshiaki, Fuchu-shi Tokyo 1830016 (JP)
(74) Representative: Gill Jennings & Every LLP
(86) International application number: PCT/JP2007/069787
(87) International publication number: WO 2008/050609

(56) References cited:
- EP-A1- 1 652 557
- JP-A- 09 108 283
- JP-A- 10 085 362
- JP-A- 2001 286 521
- JP-A- 2002 306 556
- JP-A- 2004 267 678
- JP-A- 2005 058 544
- JP-A- 2005 323 823
- JP-A- 2007 125 254
- US-A- 4 077 402
- US-A- 5 197 478
- US-A- 5 738 612
- US-A1- 2004 002 659

## Description

### Technical Field

The present invention relates to a therapeutic device that can be applied to, for example, treatment of metabolic syndrome, based on a mechanism of a training method that has been widely used in practice as a KAATSU training.

### Background of the Invention

Dr. Yoshiaki Sato, one of the inventors of the present application, has conducted studies for some time in order to work out a muscle strength increasing method for easy, safe, and effective muscle development. He has put together the accomplishments into a patent application having Japanese Patent Application No. 5-313949, which has been granted as Japanese Patent No. 2670421.

The muscle strength increasing method according to the subject patent, which involves the application of pressure, is a distinctive non-conventional one. This muscle strength increasing method (hereinafter, referred to as the "KAATSU training™ Method") is based on the following theoretical concept.

Muscles are composed of slow-twitch muscle fibers and fast-twitch muscle fibers. Slow-twitch muscle fibers are limited in their potential for growth. Accordingly, it is necessary to recruit fast-twitch muscle fibers of the slow- and fast-twitch muscle fibers in order to develop muscles. The growth hormone, which is secreted by the pituitary gland after recruitment of fast-twitch muscle fibers and the resulting lactic acid buildup in the muscles, has effects of, for example, promoting muscle growth and shedding body fat. This means that recruitment of fast-twitch muscle fibers and their fatigue result in development of fast-twitch muscle fibers and, in turn, the entire muscles.

Slow-twitch muscle fibers and fast-twitch muscle fibers are different from each other in terms of the following. Slow-twitch muscle fibers use oxygen for energy and are recruited for low-intensity activities. Fast-twitch muscle fibers provide for activities even when no oxygen is present. They are recruited after the slow-twitch muscle fibers for highly intense activities. Therefore, it is necessary to cause the earlier recruited slow-twitch muscle fibers to be exhausted soon in order to recruit fast-twitch muscle fibers.

Conventional muscle strength increasing methods use heavy exercises with, for example, a barbell to cause the slow-twitch muscle fibers to be exhausted first, and then to recruit the fast-twitch muscle fibers. Such recruitment of the fast-twitch muscle fibers requires a significant amount of exercises, is time-consuming, and tends to increase the burden on muscles and joints.

On the other hand, when muscle exercises, if done with a predetermined range of muscles near the base of the limb being compressed to restrict the blood flowing downstream away from the compressed range, reduces the amount of oxygen carried to the muscles. The slow-twitch muscle fibers, which require oxygen for energy, are thus exhausted in a short period of time. Muscle exercises with blood-flow restriction by application of a pressure will result in recruitment of the fast-twitch muscle fibers without needing a large amount of exercises. More specifically, when a predetermined range of the limb near the base thereof is compressed at an appropriate pressure, limb veins that run near the surface of the skin are occluded but the arteries which lie deeper in the limb are almost as usual. If this condition is kept for a certain period of time, the blood pumped through the arteries is blocked in the limb that has compressed near the base thereof because it cannot flow through the veins. This is quite close to the condition where the limb moves for heavy exercises, which brings about intense fatigue of the muscles. In addition, occlusion of the veins makes it hard to clear away the lactic acid that has built up in the muscles . This is another cause of the muscle fatigue.

The KAATSU Training Method can artificially produce a condition similar to the aforementioned conditions achieved during and after exercises. Thus, the KAATSU Training Method can produce effects of strength training and promote secretion of growth hormone.

Based on such a mechanism, restriction of muscle blood flow can provide significant development of the muscles.

The KAATSU Training Method is premised on the theoretical concept of muscle development by the restriction of blood flow. More specifically, an appropriate compression force is exerted on at least one of the limbs at a predetermined position near the base thereof in order to restrict the blood flowing downstream away from the compressed range. The compression force is used to put an appropriate stress attributed to the reduced blood flow on the muscles , thereby causing muscle fatigue . Thus, effective muscle development is achieved.

The KAATSU Training Method features muscle development without any exercises because it involves developing muscles by putting a stress attributed to blood flow decrease on the muscles. This feature makes the KAATSU Training Method significantly effective for restoring motor function for those who have impaired motor function such as the elderly or injured persons.

In addition, the KAATSU Training Method can compensate for a total amount of stress that is placed on the muscles by putting a stress attributed to reduced blood flow on the muscles. When combined with some exercises, the method has a feature of advantageously reducing an exercise-related stress as compared with conventional methods. This feature brings about some effects: the possibility of incurring damages to the joints or muscles can be reduced and the period of training can be reduced, as a result of decrease in amount of muscle exercises to develop the muscles.

Continued studies on the KAATSU Training Method have revealed that the KAATSU training stimulates the pituitary gland to produce much more growth hormone than normal, and that the body receives the benefits of the increasing growth hormone in addition to the increase in muscle mass. Such favorable effects provide the potential of making the KAATSU Training Method be applied to the medical field.

According to our research findings, a field where the KAATSU Training Method can be applied to a therapy is for the treatment of metabolic syndrome that has become a social problem in recent years.

Metabolic syndrome is defined as the combination of two or more of the following clinical conditions: diabetes, hypertension, and hyperlipidemia which are lifestyle diseases that tend to affect the middle-aged and elderly people. With this syndrome, a patient has a high risk factor of arteriosclerosis, myocardial infarction, or stroke, and therefore should get early treatment. According to the survey conducted by the Ministry of Health, Labour and Welfare, the number of patient with diabetes is 16.2 million (including potential cases), and there are 39 million and 22 million patients with hypertension and hyperlipidemia, respectively. In addition, it is estimated that 4.68 million people suffer from obesity which is closely related to the above clinical conditions and metabolic syndrome.

Accordingly, there are strong social demands for immediate development of a treatment method for metabolic syndrome which many people suffer.

There are generally two therapeutic approaches to the metabolic syndrome: dietary treatment and regular exercises. They are both not so easy to practice. When taking into consideration the fact that many of the metabolic syndrome patients do not exercise enough, it is particularly difficult to keep regular exercises.

Under such circumstances, it can be said that the KAATSU Training Method is exactly the right choice for the treatment of the metabolic syndrome because it advantageously produces effects similar to those produced by doing more exercises than those actually have done, or produces effects similar to those produced by doing some exercises without doing it in fact, and stimulates the secretion of more growth hormone than normal.

Based on this viewpoint, the present inventors have made studies for applying the KAATSU Training Method to the treatment of the metabolic syndrome.

As a result, the following has been learned. Since the KAATSU Training Method relies on restriction of blood flow through at least one of the limbs for its effect, at what level of pressure the limb in question is compressed is a very important factor when applied to the treatment of the metabolic syndrome, though this is not limited to cases where the method is applied to the medical field. The present inventors have proposed many techniques to control the pressure that is exerted on the limb (s) in doing the KAATSU training (Japanese Patent Application No. 8-248317, International Patent Application No. PCT/JP98/03721, Japanese Patent Application Nos. 2003-110903, 2003-169267, 2003-174813, and 2003-294014). However, it has been found that great care should be taken to control the pressure to be exerted on the limb by means of compressing a predetermined range of the limb near the base thereof when the KAATSU Training Method is intended to be applied to the treatment of the metabolic syndrome because many of the metabolic syndrome patients do not exercise enough, and, in addition, the metabolic syndrome patients who are usually middle-aged or elderly experience reduced elasticity and strength of blood vessels.

The present invention is to solve such problems and an object thereof is to provide a therapeutic device with which the KAATSU Training Method can be applied for therapies and, in particular, suitable for treating a metabolic syndrome patient.

EP-A-1652557 provides a pressure muscle strength increasing apparatus that allows accurate adjustment of the degree of blood flow restriction when a pressure muscle strength increasing method is carried out.

US4077402 discloses an apparatus for promoting blood circulation in a body part suffering from a circulation deficiency by inflating and deflating a pressure cuff applied to a patient' s limb proximal to the affected body part so that the blood is forced into the affected body part.

US5738612 is directed to an exercise apparatus including an exercise-load applying device which applies an exercise load to a person such as a patient. An exercise-load changing device which changes the exercise load applied to the person by the exercise-load applying device. A pulse-rate measuring device which successively measures a pulse rate of the person in synchronism with a heartbeat of the person. A blood-pressure measuring device which successively and non-invasively measures a blood pressure of the person in synchronism with the heartbeat of the person . A calculating device which successively calculates a product of the pulse rate and the blood pressure in synchronism with the heartbeat of the person and a control device which controls the exercise-load changing device to change the exercise load applied to the person by the exercise-load applying device, so that the products successively calculated by the calculating device substantially coincide with a predetermined target value.

JP-A-2005-323823 provides a massage machine which controls a massaging operation according to a patient condition, such as blood pressure.

US5197478 discloses an automatic sphygmomanometer arranged to predict, on the basis of the relationship between cuff pressure and the amplitude of a pulse wave, maximal and minimal blood pressures while the cuff pressure is being increased.

US 2004/0002659 A1 relates to an electronic blood pressure monitor for determining a blood pressure value using a pulse wave. At the time of measuring a blood pressure, when an MPU controls a pressure in a cuff, detects a pulse wave generated due to pressurizing of a cuff pressure via a band-pass filter or the like, calculates a wave feature value of the detected pulse wave and calculates the blood pressure so as to output it using the calculated wave feature value, the MPU detects the pulse wave in a range of the cuff pressure where the pule wave can be detected. Therefore, since a blood pressure measuring process constituted of detection of the pulse wave, calculation of the wave feature value and calculation of the blood pressure is executed within the range of the cuff pressure where the pulse wave can be detected, the process is prevented from being executed within a range where the pulse wave cannot be detected, thereby measuring the blood pressure with good accuracy.

### SUMMARY OF THE INVENTION

In order to solve the aforementioned problems, the present inventors propose the following invention.

The present inventors propose a therapeutic device as set out in claims 1 and 2.

An emobidment of the present invention is a therapeutic system comprising: a tight fitting device including a belt having the length that is enough to be wrapped around a predetermined range of muscles of one of the limbs; fastening means for fastening said belt with said belt being wrapped around said predetermined range of muscles; and a gas bag provided in or on said belt, said gas bag being adapted to apply a predetermined compression pressure to said predetermined range of muscles by means of compressing said predetermined range of muscles when said gas bag is filled with gas while said belt that has been wrapped around said predetermined range of muscles is fastened by said fastening means; together with the therapeutic device of claim 1 or claim 2.

As described above, the KAATSU training is involved in compressing or applying a pressure to a predetermined range near the base of the limb to restrict the blood flowing through the limb, thereby providing effects similar to those obtained after exercises. The aforementioned compression or application of a pressure is directed to have more blood than usual at the end of the compressed limb, by means of occluding veins in the limb while keeping arteries open, as described above.

It is noted that, when the KAATSU training is used for a healthy person, a relatively high pressure which occludes arteries to some extent maybe applied to the limb to compress a predetermined range thereon in order to completely occlude veins therein.

However, it is not appropriate to apply such a high pressure that can occlude arteries when compressing a limb of a person such as a metabolic syndrome patient whose blood vessels have lost their normal strength or elasticity. On the other hand, it is impossible to provide sufficient medical treat for a metabolic syndrome patient unless the limb of the patient is compressed by using a pressure that can occlude veins to some extent.

Taking the above into consideration, the therapeutic system according to the present invention uses preprocessing prior to normal processing for fully compressing the limb at a position near the base thereof. During the preprocessing, a maximum pulse wave pressure is determined as an appropriate pressure to be applied to the limb to compress it at a position near the base thereof. The maximum pulse wave pressure is determined based on arterial pulse wave that varies under different pressures used to compress the limb at a position near the base thereof. The term pulse wave refers to a kind of pulsation which is created when blood is ejected into the aorta by the heart during the systole and resulting changes in blood pressure propagate towards the peripheral blood vessels. Volume pulse wave is identified as changes in blood volume due to this pulsation while pressure pulse wave is identified as changes in blood pressure. In the present invention, a predetermined parameter that varies depending on either one of the changes is detected by means of pulse wave measuring means and the aforementioned maximum pulse wave pressure is determined based on it (it should be noted that, in the present invention, the predetermined parameter that varies depending on the variation of the pulse wave includes the pulse wave itself).

The reason why the aforementioned maximum pulse wave pressure is suitable as a pressure suitable for being applied to the base of the limb of a metabolic syndrome patient is as follows. The maximum pulse wave pressure is a gas pressure within the gas bag at the time point at which the changing pulse wave has reached its maximum while the pressure setting means is changing the gas pressure within the gas bag of the tight fitting device. In other words, the gas pressure within the gas bag at which the pulse wave in the limb reaches its maximum when the gas pressure within the gas bag is changed corresponds to the maximum pulse wave pressure. The maximum pulse wave means that a maximum volume of blood is being introduced through arteries to the limb that has compressed at a position near the base thereof (or the function to pump blood through arteries is maximally performed). Under such situations, the arteries are not compressed so strongly, and thus the pressure can be considered as being appropriate as the pressure to be applied to compress a limb of a metabolic syndrome patient.

The therapeutic system according to the present invention is capable of compressing the limb at a position near the base thereof during the normal processing for the actual treatment while keeping such maximum pulse wave pressure as the gas pressure within the gas bag.

Because of the aforementioned reasons, the therapeutic system according to the present invention is suitable for medical treatment for those who have weak blood vessels including metabolic syndrome patients.

As described above, the control means controls said pressure setting means during the preprocessing so that said pressure setting means changes the gas pressure within said gas bag. The change in gas pressure within the gas bag may be continuous change or stepwise change. As used herein, the expression "stepwise" means that there is a time interval during which the pressure does not change over time. The gas pressure within the gas bag may be increased with time or alternatively, it may be decreased with time. The requirement is that the gas pressure within the gas bag is changed so that the maximum pulse wave pressure can finally be determined.

As described above, the maximum pulse wave pressure is determined based on the pulse wave data. There is no limitation on how it is determined based on the pulse wave data. The pulse wave data is supplied two or more times, e.g., in a continuous manner, from the pulse wave measuring means to the control means . The pulse wave data that are continuously supplied from the pulse wave measuring means may be sent to the control means one after another without a break. Alternatively, they may be sent to the control means at constant intervals or at predetermined intervals.

When the control means controls, during said preprocessing, said pressure setting means in such a manner that said pressure setting means once raises the pressure within said gas bag until it exceeds a pressure that is expected to be higher than the maximum pulse wave pressure and thereafter reduces the pressure within said gas bag, the control means may determine the maximum pulse wave pressure in a manner as described below.

For example, said control means may be adapted to continuously receive said pulse wave data from said pulse wave measuring means while the pressure within said gas bag is decreasing during said preprocessing, and adapted to determine the maximum pulse wave pressure at the time point at which the pulse wave has reached its maximum, from at least one preceding pulse wave data (or from preceding and following pulse wave data), when said pulse wave data indicates that the amplitude of said pulse wave becomes smaller than before.

According to the studies made by the present inventors, it has been found that the pulse wave gradually grows as the pressure used to compress the limb at a position near the base thereof is reduced, and that it gradually diminishes after the pressure becomes lower than a certain pressure. Thus, as the pressure that is applied to compress the limb at a position near the base thereof is reduced, the pulse wave grows. When the pulse wave begins to diminish, it can be considered that the pulse wave has had its maximum value somewhat before the time point at which the pulse wave begins to diminish. In the aforementioned approach, when said pulse wave data indicates that the amplitude of said pulse wave becomes smaller than before, the maximum pulse wave is determined or estimated that corresponds to the maximum pulse wave pressure from at least one preceding pulse wave data (or from preceding and following pulse wave data).

In addition, said control means may be adapted to continuously receive said pulse wave data from said pulse wave measuring means while the pressure within said gas bag is decreasing during said preprocessing, and adapted to determine an immediately preceding gas pressure within said gas bag as the maximum pulse wave pressure when said pulse wave data indicates that the amplitude of said pulse wave becomes smaller than before. This approach also utilizes the nature of the pulse wave in that the pulse wave gradually grows as the pressure used to compress the limb at a position near the base thereof is reduced, and that it gradually diminishes after the pressure becomes lower than a certain pressure. When utilizing this approach, it is better to send the pulse wave data to the control means as continuous as possible or in as short a time interval as possible during the measurement of the pulse wave.

In the aforementioned two cases, it is necessary that the gas pressure within the gas bag is once increased to a level higher than a pressure that is expected as the maximum pulse wave pressure, and then the gas pressure within the gas bag is reduced. The pressure that is expected as the maximum pulse wave pressure is known, from our experience, to fall within a certain range. Therefore, it is virtually easy to do so. More specifically, the level of the gas pressure within the gas bag that should be reached once is about 230 to 250 mmHg. However, the aforementioned level of pressure that is higher than the expected maximum pulse wave pressure and that should be reached once varies among individuals. Accordingly, in this therapeutic system, it is possible to use the following configuration: the level of the gas pressure within the gas bag that should be higher than the maximum pulse wave pressure and should be reached once is allowed to be provided by using input means before the preprocessing is performed to determine the maximum pulse wave pressure, and the control means that has received the input from the input means makes the pressure setting means raise the gas pressure within the gas bag once to a pressure level based on the input.

Said control means may be adapted to control, during said preprocessing, said pressure setting means in such a manner that said pressure setting means raises a pressure within said gas bag from a pressure that is expected to be lower than the maximum pulse wave pressure, adapted to continuously receive said pulse wave data from said pulse wave measuring means while the pressure within said gas bag is increasing, and adapted to determine the maximum pulse wave pressure at the time point at which the pulse wave has reached its maximum, from at least one preceding pulse wave data, when said pulse wave data indicates that the amplitude of said pulse wave becomes smaller than before.

Alternatively, said control means may be adapted to control, during said preprocessing, said pressure setting means in such a manner that said pressure setting means raises a pressure within said gas bag from a pressure that is expected to be lower than the maximum pulse wave pressure, adapted to continuously receive said pulse wave data from said pulse wave measuring means while the pressure within said gas bag is increasing, and adapted to determine an immediately preceding gas pressure within said gas bag as the maximum pulse wave pressure when said pulse wave data indicates that the amplitude of said pulse wave becomes smaller than before.

According to the studies made by the present inventors, it has been found that the pulse wave gradually grows as the pressure used to compress the limb at a position near the base thereof raises, and that it gradually diminishes after the pressure becomes higher than a certain pressure. Thus, these two approaches also can be used to determine the maximum pulse wave pressure as in the case in which the pressure to compress the limb at a position near the base thereof is reduced. When these two approaches are performed, it is necessary that the gas pressure within the gas bag is reduced to a level lower than a pressure that is expected as the maximum pulse wave pressure, and then the gas pressure within the gas bag is increased. This can be achieved by means of, for example, increasing the gas pressure within the gas bag from the normal pressure.

The parameter that varies depending on the variation of the amplitude of the arterial pulse wave and is measured by the pulse wave measuring means may be any physical quantity. The pulse wave measuring means may be, for example, a sensor that measures a surface pressure on the skin when being pressed against the skin surface. It may be a sensor that measures a surface pressure from the skin based on the pulse wave. The pulse wave is observed on the skin surface as pulsation, so that the aforementioned pulse wave measuring means measures, as the parameter, the surface pressure on the skin that varies depending on the pulsation.

Alternatively, the pulse wave measuring means may be capable of measuring the gas pressure within said gas bag as said parameter. As described above, the pulse wave is observed on the skin surface as pulsation. The pulsation causes the air pressure to change within the gas bag of the tight fitting device that has been wrapped around the limb at a position near the base thereof. The aforementioned pulse wave measuring means measures this air pressure within the gas bag as the parameter.

It should be noted that the pulse wave measuring means needs only be able to measure a pulse wave at a position near said predetermined range of muscles or a position closer to the distal end of the limb than there. The pulse wave measuring means is not necessarily required to be the one that can measure a pulse wave at a position closer to the distal end of the limb than the predetermined range, when the pulse wave measuring means measures a pulse wave at a position near the predetermined range of muscles.

The therapeutic system according to the present invention may comprise a single tight fitting device or, alternatively, two or more tight fitting devices.

When two or more tight fitting devices are included, said pulse wave measuring means are equal in number to said tight fitting devices and associated with their respective one of said tight fitting devices. Said pulse wave measuring means may be adapted to measure a predetermined parameter that varies depending on the variation of the amplitude of the pulse wave, at a position near the predetermined range of muscles around which the associated tight fitting device is wrapped, or a position closer to the distal end of the limb than there, and to generate a pulse wave data associated with the limb. In addition, said pressure setting means in this case are equal in number to said tight fitting devices and may be associated with their respective one of said tight fitting devices. Furthermore, said control means in this case may be adapted to determine, during said preprocessing, the maximum pulse wave pressure for each of the limb, and adapted to control, during said normal processing, each of said pressure setting means associated with said tight fitting device that compresses one of the limbs, so that each of said pressure setting means sets the gas pressure within each gas bag included in the tight fitting device associated with the pressure setting means to said maximum pulse wave pressure. In this case, the maximum pulse wave pressures to be determined for the respective tight fitting devices may be different from one tight fitting device to another . By using such a therapeutic system, two or more tight fitting devices can be used to independently control the compression pressure exerted on each of the limbs.

In a comparative example the following method carried out by the therapeutic device is provided.

The method is a control method that is carried out by a therapeutic device that forms a therapeutic system by means of being combined with a tight fitting device including a belt having the length that is enough to be wrapped around a predetermined range of muscles of one of the limbs; fastening means for fastening said belt with said belt being wrapped around said predetermined range of muscles; and a gas bag provided in or on said belt, said gas bag being adapted to apply a predetermined compression pressure to said predetermined range of muscles by means of compressing said predetermined range of muscles when said gas bag is filled with gas while said belt that has been wrapped around said predetermined range of muscles is fastened by said fastening means; said therapeutic device comprising: pressure setting means which is capable of setting a gas pressure within said gas bag to a predetermined pressure; control means for controlling said pressure setting means in order to change said compression pressure; and pulse wave measuring means for measuring a predetermined parameter that varies according to the variation of amplitude of arterial pulse wave that varies depending on said compression pressure, at a position near said predetermined range of muscles or a position closer to the distal end of the limb than there, to generate a pulse wave data associated with the parameter.

In this method, said control means makes said pressure setting means execute two processing, i.e., preprocessing and normal processing; controls said pressure setting means during said preprocessing so that said pressure setting means changes the gas pressure within said gas bag, and determines a maximum pulse wave pressure that is a gas pressure within said gas bag at the time point at which the amplitude of the pulse wave has reached its maximum, by means of receiving two or more said pulse wave data from said pulse wave measuring means while the pressure within said gas bag is changing; and controls said pressure setting means during said normal processing so that said pressure setting means sets the gas pressure within said gas bag to said maximum pulse wave pressure.

### BRIEF DESCRIPTION OF THE DRAWINGS

Fig. 1 is a view schematically showing the entire configuration of a therapeutic system according to one embodiment of the present invention;
Fig. 2 is a perspective view showing a tight fitting device included in the therapeutic system shown in Fig. 1;
Fig. 3 is a view illustrating how a tight fitting device for arms included in the therapeutic system shown in Fig. 1 is used;
Fig. 4 is a view illustrating how a tight fitting device for legs included in the therapeutic system shown in Fig. 1 is used;
Fig. 5 is a view schematically showing an internal structure of a body segment included in the therapeutic system shown in Fig. 1;
Fig. 6 is a hardware configuration of a control segment included in the therapeutic system shown in Fig. 1;
Fig. 7 is a view showing a functional block generated within the control segment included in the therapeutic system shown in Fig. 1;
Fig. 8 is a view illustrating a transition of the amplitude of pulse wave while an air pressure within a gas bag is changing;
Fig. 9 is a view illustrating a transition of the amplitude of pulse wave while an air pressure within a gas bag is changing;
Fig. 10 is a view schematically showing an internal structure of a body segment included in the therapeutic system according to a second embodiment.

### BEST MODE FOR CARRYING OUT THE INVENTION

Referring to the drawings, preferred first and second embodiments of the present invention are described. In the following description of the embodiments, similar components and parts are depicted by the like reference numerals, and redundant description will be omitted as the case may be.

### <<First Embodiment>>

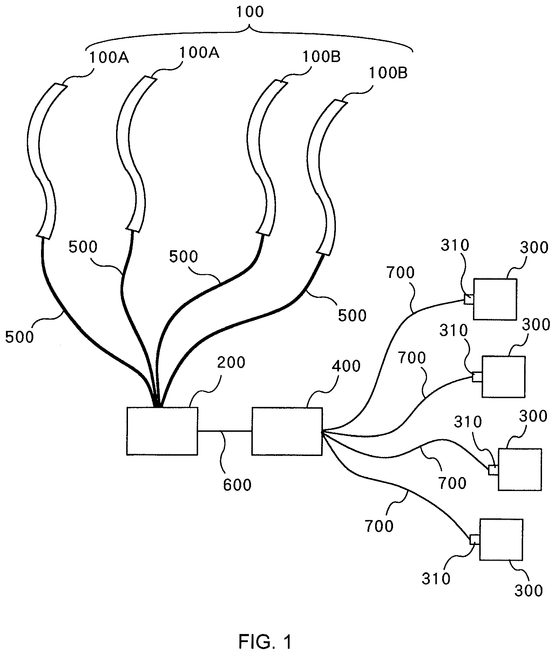

Fig. 1 is a view schematically showing the entire configuration of a therapeutic system according to one embodiment of the present invention. This therapeutic system is suitable for the treatment of the metabolic syndrome.

As shown in Fig. 1, a therapeutic system according to this embodiment is comprised of a tight fitting device 100, a body segment 200, measuring segments 300, and a control segment 400. It should be noted that a combination of the body segment 200, the measuring segments 300, and the control segment 400 corresponds to the therapeutic device according to the present invention. While the body segment 200 and the control segment 400 in this embodiment are described as separate segments, they can be integrated to each other as a single segment.

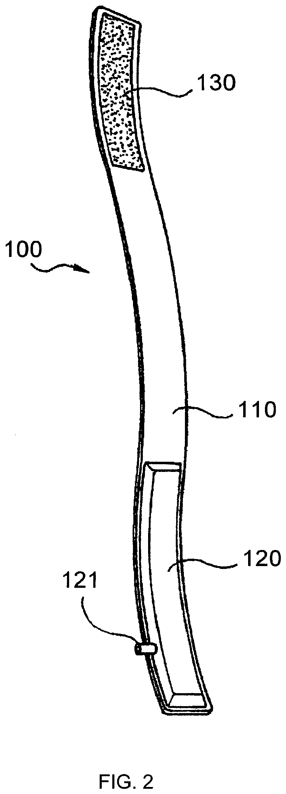

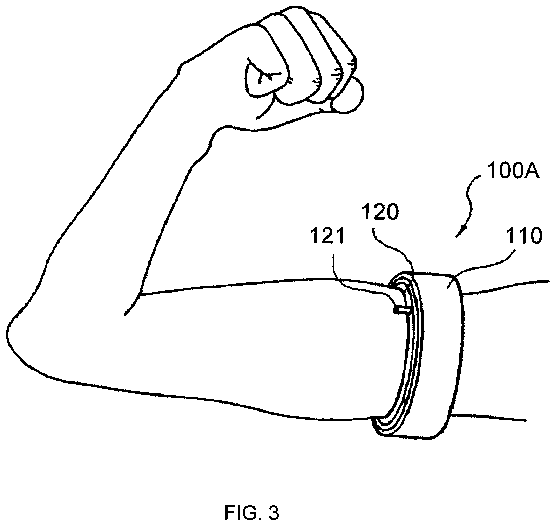

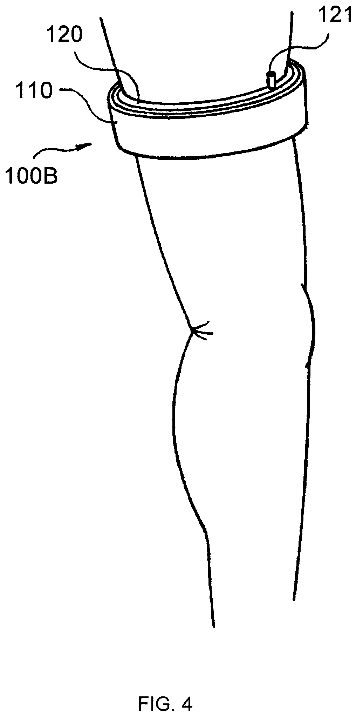

The tight fitting device 100 in this embodiment is configured as shown in Figs. 2, 3, and 4. Fig. 2 is a perspective view showing an embodiment of the tight fitting device 100. Figs . 3 and 4 are perspective views illustrating how the tight fitting device 100 is used.

The tight fitting device 100 in this embodiment comprises a plurality of, more specifically, four members as shown in Fig. 1. The reason why there are four tight fitting devices 100 is to allow compression of both arms and both legs of a patient who receives medical treatment for metabolic syndrome. Of the tight fitting devices 100 in this embodiment, tight fitting devices 100A are for arms (that are intended to be wrapped around an arm for the compression of the arm) while tight fitting devices 100B are for legs (that are intended to be wrapped around a leg for the compression of the leg). The number of the tight fitting devices 100 is not necessarily four. Any number equal to or larger than one may be used. The number of the tight fitting device(s) 100A for arms is not necessarily identical with the number of the tight fitting device(s) 100B for legs.

The tight fitting device 100 in this embodiment is for compressing a predetermined range of the limb near the base thereof with a predetermined pressure while being rest on one of the limb near the base thereof. The tight fitting device 100 is designed so that the pressure to be applied to the predetermined range of the limb near the base thereof can be varied. This tight fitting device 100 basically comprises a belt 110, a gas bag 120, and a fastening member 130 in this embodiment.

Any belt may be used as the belt 110 as long as it can be wrapped around a predetermined range of the limb near the base thereof (more specifically, located at a position near the top of the arm or near the top of the leg that is suitable for the restriction of the blood flow by the external compression; which is hereinafter referred to as a "range to be compressed") which the tight fitting device 100 is wrapped around.

The belt 110 in this embodiment may be made of a stretchable material but there may be no need to do so. For example, the belt 110 is formed of a polyvinyl chloride (PVC).

The length of the belt 110 according to this embodiment is determined in accordance with the circumferential length of the range to be compressed of a person who receives medical treatment for metabolic syndrome. The length of the belt 110 may be any length that is longer than the circumferential length of the range to be compressed. The length of the belt 110 in this embodiment is twice or longer than the circumferential length of the range to be compressed. The length of the belt 110 of the tight fitting device 100A for arms according to this embodiment is determined in view of the circumferential length of the range to be compressed on the arm being 26 cm. More specifically it is 90 cm. The length of the belt 110 of the tight fitting device 100B for legs is determined in view of the circumferential length of the range to be compressed on the leg being 45 cm. More specifically, it is 145 cm.

The width of the belt 110 according to this embodiment may suitably be determined depending on the exact position of the range to be compressed by the tight fitting device 100. For example, the belt 110 of the tight fitting device 100A for arms of which the range to be compressed is located near the top of the arm may be about 3 cm in width while the belt 110 of the tight fitting device 100B for legs of which the range to be compressed is located near the top of the leg may be about 5 cm in width.

The gas bag 120 is attached to the belt 110. The gas bag 120 in this embodiment is attached to one surface of the belt 110. However, the way to attach the gas bag 120 to the belt 110 is not limited thereto. The gas bag 120 may be provided within the belt 110 having a shape of a hollow cylinder.

One end of the gas bag 120 is aligned with the corresponding end of the belt 110 (the lower end of the belt 110 in Fig. 2) but there may be no need to do so. The gas bag 120 is an air-tight bag. The gas bag 120 in this embodiment is made of a stretchable rubber similar to that of, for example, an inflatable bladder used in a blood pressure cuff. The material of the gas bag 120 is not limited thereto. Any material that can maintain air tightness may appropriately be used.

The length of the gas bag 120 is, in this embodiment, generally equal to the circumferential length of the range to be compressed but there may be no need to do so. In this embodiment, the gas bag 120 of the tight fitting device 100A for arms is 25 cm in length while the gas bag 120 of the tight fitting device 100B for legs is 45 cm in length.

The width of the gas bag 120 may suitably be determined depending on the exact position of the range to be compressed by the tight fitting device 100. In this embodiment, the gas bag 120 of the tight fitting device 100A for arms is about 3 cm in width while the gas bag 120 of the tight fitting device 100B for legs is about 5 cm in width but there may be no need to do so.

The gas bag 120 has a connection inlet 121 that is communicated with the inside of the gas bag 120. It may be connected with the body segment 200 through, for example, a connecting pipe 500 comprised of a rubber tube. As will be described below, through the connection inlet 121, a gas (air in this embodiment) is introduced into the gas bag 120 or the gas in the gas bag 120 is vented to the outside.

The fastening member 130 is for fastening the belt 110 so that it is held with being wrapped around the range to be compressed. The fastening member 130 in this embodiment is a two-dimensional fastener provided at the other end of the belt 110 (the upper end of the belt 110 in Fig. 2) on the side of the belt 110 on which the gas bag 120 is provided. The fastening member 130 can be fastened to any part of the entire surface of the belt 110 on which the gas bag 120 is not provided.

When the gas bag 120 is filled with air after the belt 110 is wrapped around the range to be compressed and the belt 110 is fastened by using the fastening member 130, the tight fitting device 100 compresses the range to be compressed at an appropriate pressure. On the other hand, removal of the air from the gas bag 120 at that state reduces the pressure to compress the range to be compressed by the tight fitting device 100.

The body segment 200 is designed in such a manner that it can supply a gas to the gas bag 120 and remove the gas from the gas bag 120. As long as it can supply a gas to the gas bag 120 and remove the gas from the gas bag 120, any one of possible configurations may be used for these purposes.

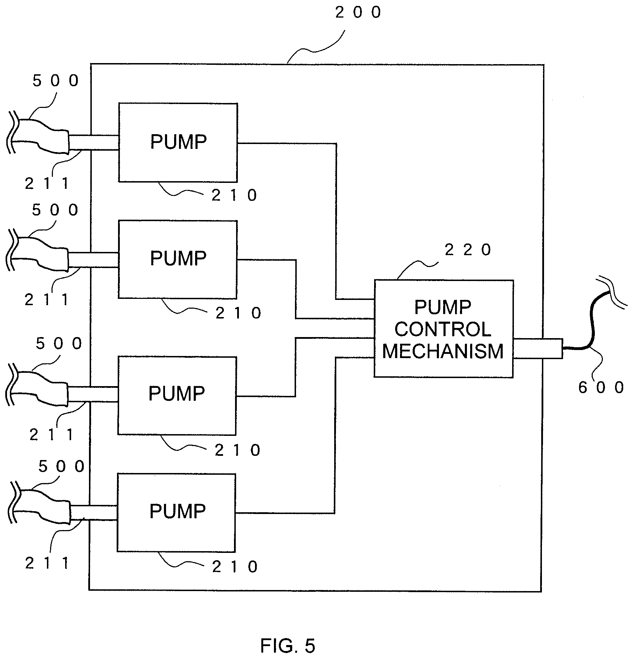

Fig. 5 schematically shows a structure of an exemplified body segment 200. As shown in Fig. 5, the body segment 200 is composed of four pumps 210 and a pump control mechanism 220. These four pumps 210 are connected to four tight fitting devices 100, respectively, via the connecting pipes 500 and are associated with the respective tight fitting devices 100 connected thereto via the respective connecting pipes 500.

The pump 210 has a function of sucking the surrounding gas (air in this embodiment) and supplying it to the outside of a pump connection inlet 211 which will be described below. It includes a valve which is not shown. To open the valve results in removal of the gas in the pump 210 to the outside. Each of the four pumps 210 has its own pump connection inlet 211 and is connected to the gas bag 120 through the connecting pipe 500 connected thereto and the connection inlet 121. When the pump 210 forces the gas, the gas is introduced into the gas bag 120 of the tight fitting device 100 associated with that pump 210. When the pump 210 opens the valve, the gas can be removed from the gas bag 120 of the tight fitting device 100 associated with that pump 210.

As will be described later, the pump control mechanism 220 controls the pumps 210 according to the data received from the control segment 400 to make the pump(s) 210 introduce the gas into the gas bag(s) 120 or make the pump(s) 210 remove the gas from the gas bag(s) 120. In order to receive the data, the body segment 200 is connected to the control segment 400 via a cable 600 having one end connected to a terminal that the pump control mechanism 220 comprises.

The measuring segment 300 is adapted to measure arterial pulse wave from the limb that varies depending on the compressing pressure when the tight fitting device 100 compresses a predetermined range to be compressed of the limb at a position near the range to be compressed of the limb around which the tight fitting device 100 is wrapped or a position closer to the distal end than there, while the tight fitting device 100 is rest on the range to be compressed of the limb.

The measuring segment 300 in this embodiment comprises four members, as in the case of the tight fitting devices 100. The four measuring segments 300 are associated with one of the tight fitting devices 100. This means that the therapeutic system of this embodiment includes four pairs of the tight fitting device 100 and the measuring segment 300.

The measuring segment 300 in this embodiment is adapted to measure pulse wave as described above and produce a pulse wave data indicating the measured pulse wave. The pulse wave measured by the measuring segment 300 may be volume pulse wave or pressure pulse wave. The measuring segment 300 in this embodiment measures the pressure pulse wave. The measuring segment 300 for measuring the pressure pulse wave is, in this embodiment, implemented by using a pressure sensor that can measure a surface pressure. When the pulse wave to be measured is the volume pulse wave, the measuring segment 300 may be, for example, comprised of a phototransistor that is used for photoelectric plethysmogram.

The measuring segment 300 in this embodiment is capable of successively and continuously measuring, without a break, a predetermined parameter that varies depending on the variation of the amplitude of the pulse wave, but there may be no need to do so. In other words, the measuring segment 300 can continuously measure a predetermined parameter that varies depending on the variation of the possibly ever-changing amplitude of the pulse wave. It should be noted that the measuring segment 300 may be the one adapted to measure a predetermined parameter that varies depending on the variation of the amplitude of the pulse wave at a predetermined time interval, such as every 30 seconds.

All of the four measuring segments 300 measure the amplitude of the pulse wave and produce a pulse wave data regarding the aforementioned parameter to send it to the control segment 400. In order to make this possible, the measuring segment 300 comprises an output terminal 310 (see Fig. 1) and is adapted to send the pulse wave data to the control segment 400 through a cable 700 connected at one end thereof to the output terminal 310. The other end of the cable 700 is connected to the control segment 400. The configuration to send the pulse wave data to the control segment 400 is not limited thereto. For example, data may be sent wirelessly to the control segment 400 by using a light beam or radio wave. In this embodiment, the pulse wave data generated by the measuring segment 300 based on the parameters that have measured one after another without a break is sent to the control segment 400 almost in real time.

It should be noted that the measuring segment 300 in this embodiment may be integrated with the tight fitting device 100 as a single unit.

The control segment 400 is for controlling the body segment 200. More specifically, the control segment 400 produces data for controlling each of the four pumps 210 in the body segment 200 and sends the data to the pump control mechanism 220, thereby making the pump control mechanism 220 control the pumps 210.

In addition, the control segment 400 comprises an external input device which is not shown. The input device is a known input device including numeric keypads.

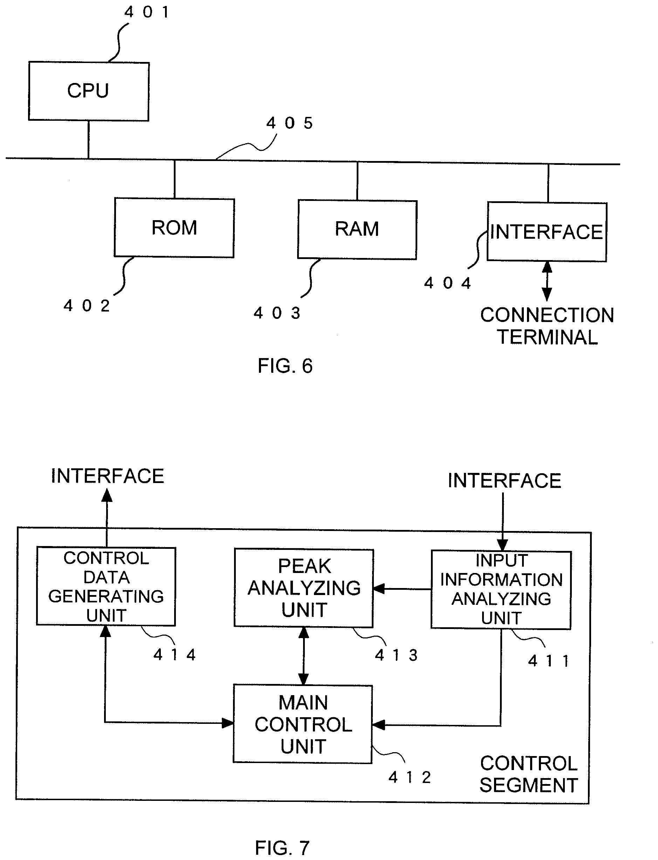

The internal configuration of the control segment 400 is schematically shown in Fig. 6. The control segment 400 contains a computer. A CPU 401, an ROM 402, an RAM 403 and an interface 404 are connected to each other through a bus 405.

The CPU 401 is a central processing unit and is for controlling the entire control segment 400. The ROM 402 records a program and data that are necessary for the below-described processing which is to be carried out by the control segment 400. The CPU 401 executes the processing described below based on the program. The ROM 402 may be embodied by using a flash ROM. The RAM 403 is for providing a working area for the execution of the aforementioned program. The interface 404 is a device for the exchange of data between the outside. In addition to the ROM 402 and the RAM 403, a hard disk may be provided as a component that can provide similar functions to them.

The interface 404 is connected to a connection terminal (not shown) that can be connected to one end of the cable 600, and four connection terminals (not shown) that can be connected to the other end of the cable 700. The aforementioned pulse wave data supplied from the measuring segment 300 is received by the interface 404 through the cable 700. In addition, the control data described below is sent from the interface 404 to the body segment 200 through the cable 600. The interface 404 is connected to the aforementioned input device and receives data generated in response to an operation of the input device.

As the CPU 401 executes the aforementioned program, functional blocks as shown in Fig. 7 are created within the control segment 400.

The control segment 400 includes an input information analyzing unit 411, a main control unit 412, a peak analyzing unit 413, and a control data generating unit 414.

The input information analyzing unit 411 receives the pulse wave data or data from the input device through the interface 404 and analyzes details thereof. When the data received by the input information analyzing unit 411 is the pulse wave data, it is transferred to the peak analyzing unit 413 without any modification. When the data received by the input information analyzing unit 411 is data from the input device, data representing the result of analysis by the input information analyzing unit 411 is supplied to the main control unit 412.

The main control unit 412 is for controlling the entire control segment 400.

The main control unit 412 firstly performs control for selecting and executing two modes to be carried out in this therapeutic system. In this therapeutic system, operations may be performed in one of two modes: an automatic mode and a manual mode.

The automatic mode can be understood in two types of processing: preprocessing and normal processing.

The operation in automatic mode is performed when an input to choose the automatic mode is made with the input device. The automatic mode is a mode for automatically determining the gas pressure within the gas bag 120 in treatment to perform treatment.

When an input to choose the automatic mode is made with the input device, data indicating that is supplied to the input information analyzing unit 411 through the interface 404. The input information analyzing unit 411 analyzes details of the data and sends them to the main control unit 412. Thus, an operation is performed in the automatic mode. In this case, the main control unit 412 generates data associated with an instruction to perform an operation in the automatic mode and sends it to the control data generating unit 414 and the peak analyzing unit 413.

Details of the automatic mode will be described later.

Next, the manual mode is described. An operation in the manual mode is performed when an input to choose the manual mode is made with the input device. The manual mode is a mode for manually determining the gas pressure within the gas bag 120 in treatment to perform treatment.

When the manual mode is selected, received are data indicating information to show that the manual mode is selected, along with or followed by data indicative of information about what level of pressure is used within a gas bag 120 of which tight fitting device 100 or about how long the pressure is kept. These data are supplied from the input device to the input information analyzing unit 411 through the interface 404. The input information analyzing unit 411 analyzes details of these data and sends them to the main control unit 412. Thus, an operation is performed in the manual mode. In this case, the main control unit 412 generates data to indicate that the operation is performed in the manual mode and sends it to the control data generating unit 414 along with the data indicating the information about what level of pressure is used within the gas bag 120 of which tight fitting device 100 and about how long the pressure is kept.

It should be noted that the data indicating the information about what level of pressure is used within the gas bag 120 of which tight fitting device 100 and about how long the pressure is kept may be different from one tight fitting device 100 to another. The data indicating the information about what level of pressure is used within the gas bag 120 of which tight fitting device 100 and about how long the pressure is kept does not need to indicate that the pressure within the gas bag 120 is kept constant. Instead, it may indicate that the pressure within the gas bag 120 is varied over time.

If the data indicative of performing an operation in the automatic mode is supplied from the main control unit 412, the peak analyzing unit 413 receives the pulse wave data received through the cable 700, the interface 404, and the input information analyzing unit 411 when preprocessing in the automatic mode is performed. Then, based on the pulse wave data, it detects the time point at which the amplitude of the pulse wave has reached its maximum during the preprocessing in the automatic mode, in generally real time. Details about how the time point at which the amplitude of the pulse wave has reached its maximum is detected will be described below.

The control data generating unit 414 is for generating a control data that is used to control the body segment 200 based on the data received from the main control unit 412. The control data generating unit 414 is adapted to supply the generated control data to the body segment 200 through the interface 404. It should be noted that the control data is also supplied to the main control unit 412 as a case may be, as described below.

How the control data generating unit 414 generates the control data will be described later. The pump control mechanism 220 in the body segment 200 that receives it controls each of the pump 210 based on the control data.

Next, how the therapeutic system is used is described.

In order to treat a patient having metabolic syndrome by using this therapeutic system, the four tight fitting devices 100 are wrapped around a range to be compressed on the limb of a patient. The two tight fitting devices 100A for arms are rest on the arms and the two tight fitting devices 100B for legs are rest on the legs. More specifically, the gas bag 120 is encircled once around the range to be compressed, and the excessive length of the belt 110 is further encircled two times around it. Then , the fastening member 130 is used to fasten the end of the belt 110.

Next, the four measuring segments 300 are attached at positions suitable for taking pulse waves in the arms and legs on which the four tight fitting devices 100 are rest, respectively (more exactly, pulse waves at a position near the range to be compressed of the limb or a position closer to the distal end than there). In this embodiment, each measuring segment 300 is attached at a position closer to the distal end of the limb than the tight fitting device 100 so that it contacts with the tight fitting device 100.

Next, the four tight fitting devices 100 are connected to the body segment 200 through the respective connecting pipes 500. The four measuring segments 300 are connected to the control segment 400 through the respective cables 700. The control segment 400 and the body segment 200 are connected to each other through the cable 600.

With this state, treatment of the metabolic syndrome is initiated.

At the beginning of the treatment, a person who provides the treatment, such as a clinician, operates the input device to choose either the automatic mode or the manual mode.

In the following, for the purpose of simplicity, description is made for a case where only one of the limbs is to be treated. In practice, the treatment as described below can be applied to two or more of the limbs. In the case of this embodiment, the tight fitting devices 100 are attached to all of the limbs of the patient, so two or more limbs should be treated. When two or more of the limbs are to be treated, each limb may be separately treated one by one without any temporal overlap. Two or more limbs may be treated simultaneously or, alternatively, two or more limbs may be treated at slightly different times with some overlap.

When the automatic mode is selected, the data indicative of that is supplied through the interface 404 and the input information analyzing unit 411 to the main control unit 412. The main control unit 412 sends data indicative of performing an operation in the automatic mode, to the peak analyzing unit 413 and the control data generating unit 414. The peak analyzing unit 413 and the control data generating unit 414 which have received the data begin the preprocessing in the automatic mode.

For the preprocessing, the control data generating unit 414 generates a control data. The control data generating unit 414 sends the generated control data to the pump control mechanism 220 in the body segment 200 and the main control unit 412, through the interface 404 and the cable 600. The control data supplied to the pump control mechanism 220 is to let the pump 210 immediately (such as within one second) introduce the air into the gas bag 120 until the air pressure within the gas bag 120 obviously exceeds the maximum pulse wave pressure that is the air pressure within the gas bag 120 at the time the amplitude of the pulse wave has reached its maximum (e.g., approximately 1.5 to 2.0 times higher than the pressure expected as the maximum pulse wave pressure) and to let the pump 210 reduce the air pressure within the gas bag 120 over about 5 seconds until the air pressure within the gas bag 120 obviously becomes lower than the maximum pulse wave pressure (e.g. , approximately 0.5 to 0.7 times lower than the pressure expected as the maximum pulse wave pressure).

The pump control mechanism 220 that has received the data lets the pump 210 be driven according to that data. Thus, the pump 210 forces the air into the gas bag 120 of the tight fitting device 100 associated with the subject pump 210 and then opens a valve to vent the air from the gas bag 120. As a result, the air pressure within the gas bag 120 of the tight fitting device 100 rises once to a significantly high level. The pressure exerted by the tight fitting device 100 on the range to be compressed also rises once to a significantly high level. Thereafter, the air pressure within the gas bag 120 of the tight fitting device 100 and the pressure exerted by the tight fitting device 100 on the range to be compressed are both decreased. It should be noted that the pressure within the gas bag 120 may be decreased either continuously or stepwise (when there is a time interval during which the pressure does not change over time).

On the other hand, during the preprocessing, the pressure exerted by the tight fitting device 100 on the range to be compressed in order to compress the range to be compressed varies , which fluctuates the pulse wave accordingly. The measuring segments 300 continuously measures over time a predetermined parameter that varies depending on the variation of the thus-changing amplitude of the pulse wave (in this embodiment, this parameter is a pressure given to the measuring segment 300 from the skin which varies depending on the variation of the pulse wave) . It then generates a pulse wave data indicative of that parameter and sends it to the input information analyzing unit 411 through the cable 700 and the interface 404. The peak analyzing unit 413 which receives them without a break determines exactly when the amplitude of the pulse wave has reached its maximum, based on the pulse wave data.

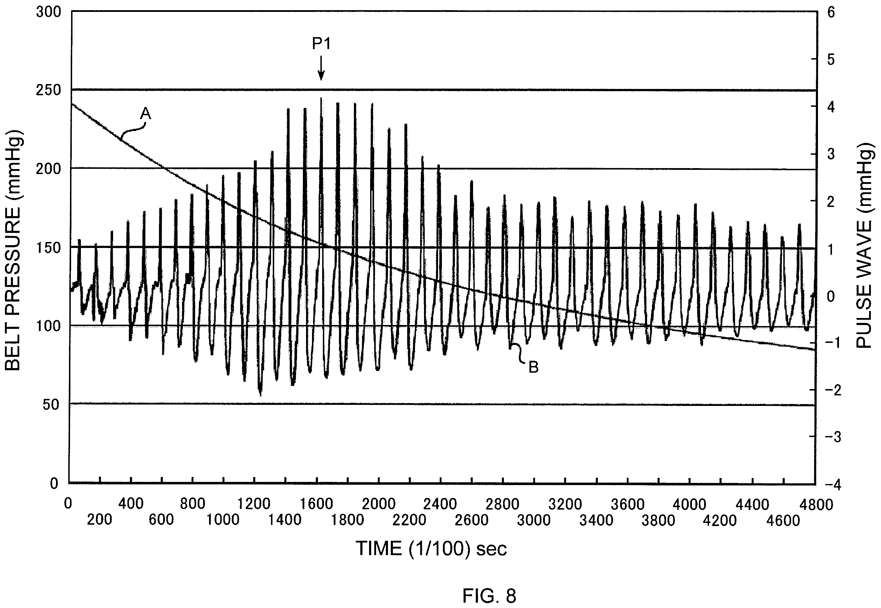

The peak analyzing unit 413 determines the time point at which the amplitude of the pulse wave has reached its maximum, in a manner described below. Fig. 8 shows an example of a measured pulse wave. A gentle downward-sloping curve from left to right, which is depicted by a symbol A in the figure, represents a gas pressure (in mmHg) within the gas bag 120. On the other hand, a waveform which is depicted by a symbol B in the figure represents the amplitude of the pulse wave (in mmHg) . More specifically, the amplitude of the pulse wave gradually increases as the pressure exerted by the tight fitting device 100 on the range to be compressed in order to compress the range to be compressed decreases. The amplitude of the pulse wave begins to decrease when the pressure exerted by the tight fitting device 100 on the range to be compressed in order to compress the range to be compressed becomes lower than a certain pressure. The amplitude of the pulse wave is determined as the peak to peak amplitude of the waveform. In Fig. 8, the amplitude of the pulse wave turns from increase to decrease at the time point depicted by a symbol P1 in the figure. The peak analyzing unit 413 continuously monitors the amplitude of the pulse wave according to the pulse wave data, as described above. It defines the time point at which the amplitude of the pulse wave turns from increase to decrease, as the time point at which the amplitude of the pulse wave has reached its maximum. It should be noted that the peak analyzing unit 413 may be adapted to define (estimate) the time point at which the pulse wave reaches its maximum by using at least one data indicative of the amplitude of the pulse wave before or before and after the time point depicted by the symbol P1 in the figure, e.g., may be adapted to define (estimate) the time point at which the pulse wave reaches its maximum by differentiating a function about the amplitude of the pulse wave with respect to time to obtain the time point at which the amplitude of the pulse wave reaches its relative maxima, and define (estimate) the time point obtained, as the time point at which the pulse wave reaches its maximum.

At any rate, the peak analyzing unit 413 generates the data indicative of the time point at which the amplitude of the pulse wave has reached its maximum and sends it to the main control unit 412. As described above, the main control unit 412 receives the control data to be used for the preprocessing from the control data generating unit 414. Accordingly, by comparing the variation of the air pressure within the gas bag 120 that can be determined using the control data with the time point at which the amplitude of the pulse wave that can be determined using the data received from the peak analyzing unit 413 has reached its maximum, the main control unit 412 defines the air pressure within the gas bag 120 at the time point at which the amplitude of the pulse wave has reached its maximum, as the maximum pulse wave pressure.

This completes the preprocessing.

Next, the main control unit 412 sends a data to the control data generating unit 414 to direct the latter to perform the normal processing. The main control unit 412 also sends to the control data generating unit 414 a data indicative of the maximum pulse wave pressure that corresponds to the air pressure within the gas bag 120 during the normal processing.

Upon being directed to perform the normal processing, the control data generating unit 414 generates a control data and sends it to the pump control mechanism 220 in the body segment 200 through the interface 404 and the cable 600. The control data is for directing to drive the pump 210 in such a manner that the air pressure within the gas bag 120 reaches the maximum pulse wave pressure and that state can be kept for a predetermined period of time (often 10 to 15 minutes for the treatment on an arm, and often 15 to 20 minutes for the treatment on a leg).

Upon reception of the control data, the pump control mechanism 220 drives the pump 210 in accordance with the instruction by the control data. This makes the tight fitting device 100 keep the air pressure within the gas bag 120 at the maximum pulse wave pressure for a predetermined period of time. In this way, according to this therapeutic system, metabolic syndrome can be treated in a safe and effective manner.

After the lapse of a certain period of time, the pump 210 opens the valve to remove the air within the gas bag 120. It should be noted that a lamp or an alarm as a means to notify, for example, a patient or a clinician that treatment has finished may be provided anywhere in the therapeutic system and completion of the treatment can be indicated to them by turning on the lamp or sounding the alarm.

When the manual mode is selected, a clinician for example provides, with the input device, the data indicative of the selection of the manual mode and the data indicative of information about what level of pressure is used within the gas bag 120 of the tight fitting device 100 and about how long the pressure is kept. These data are received by the input information analyzing unit 411 where the details of the data are analyzed. The input information analyzing unit 411 sends the result of analysis to the main control unit 412.

Upon reception of the data, the main control unit 412 sends the data to the control data generating unit 414 along with the data associated with an instruction to perform an operation in the manual mode. Upon reception of the data associated with the instruction, the control data generating unit 414 generates a control data indicative of an instruction to let the pump 210 be driven in such a manner that the pressure within the gas bag 120 of the tight fitting device 100 is kept for a given period of time, according to that instruction as well as the data that has received from the main control unit 412. The generated control data is sent to the pump control mechanism 220 through the interface 404 and the cable 600.

The pump control mechanism 220 that has received this control data let the pump 210 be driven according to the instruction by the control data. As a result, the air pressure within the gas bag 120 of the tight fitting device 100 varies in accordance with the conditions given by, for example, the clinician. In such a case, it is possible that the air pressure within the gas bag 120 exceeds the aforementioned maximum pulse wave pressure. However, safety and effectiveness of the treatment can be ensured as long as the input device is manipulated by a person who has expert knowledge such as a clinician.

After the lapse of a certain period of time, the pump 210 opens the valve to remove the air within the gas bag 120. It should be noted that completion of the treatment can be indicated to a patient or a clinician by turning on the lamp or sounding the alarm, as described above.

When a treatment is performed in the automatic mode or the manual mode, the patient may keep still or do light exercises .

In addition, the preprocessing in the automatic mode may be performed only during the first time when a patient uses this therapeutic system for his or her treatment and this may be skipped in the second treatment and later by means of using the maximum pulse wave pressure that has determined during the first treatment. However, the maximum pulse wave pressure may vary depending on, for example, health condition of the patient. Thus, it is preferable that the preprocessing be performed to determine the maximum pulse wave pressure each time when this therapeutic system is used for treatment.

### <Modified Version>

Although the therapeutic system according to the first embodiment is as described above, the preprocessing in the therapeutic system according to the first embodiment may be modified as follows. Briefly, in the preprocessing performed when the automatic mode is selected, the therapeutic system according to the first embodiment once raises the gas pressure within the gas bag 120 up to a pressure that is expected to be obviously higher than the maximum pulse wave pressure, and thereafter it reduces the pressure. However, in this modified version, the pressure is controlled so that it gradually increases from the normal pressure. It should be noted that the therapeutic system in this modified version is not different in hardware configuration from the therapeutic system according to the first embodiment.

Now, an operation in the automatic mode performed in the modified version is described.

In the modified version, when the automatic mode is selected, the data indicative of it is supplied through the interface 404 and the input information analyzing unit 411 to the main control unit 412. The main control unit 412 sends a data indicative of performing an operation in the automatic mode, to the peak analyzing unit 413 and the control data generating unit 414. The peak analyzing unit 413 and the control data generating unit 414 that have received this data begin the preprocessing in the automatic mode.

For the preprocessing, the control data generating unit 414 generates a control data. In the modified version, the control data is different from the one in the first embodiment.

In the modified version, the control data is for directing the pump 210 to raise the air pressure within the gas bag 120 from the normal pressure to a pressure that is obviously higher than the maximum pulse wave pressure (e.g., approximately 1.5 to 2.0 times higher than the pressure expected as the maximum pulse wave pressure) in an appropriate period of time (e.g., in five seconds) and then reduce the air pressure within the gas bag 120 back to the normal pressure. The control data generating unit 414 sends the generated control data to the pump control mechanism 220 in the body segment 200 and the main control unit 412 through the interface 404 and the cable 600.

The pump control mechanism 220 that has received the data lets the pump 210 be driven according to that data. Thus, the pump 210 forces the air into the gas bag 120 of the tight fitting device 100 associated with that pump 210 to raise the air pressure within the gas bag 120 to a pressure that is obviously higher than the maximum pulse wave pressure, and then removes the air from the gas bag 120.

While the pressure within the gas bag 120 is high enough, the pressure exerted by the tight fitting device 100 on the range to be compressed in order to compress the range to be compressed varies, which fluctuates the pulse wave accordingly. The measuring segment 300 continuously measures over time a predetermined parameter that varies depending on the variation of the thus-changing amplitude of the pulse wave. It then generates a pulse wave data indicative of that parameter and sends it to the input information analyzing unit 411 through the cable 700 and the interface 404. The peak analyzing unit 413 which receives them without a break determines the time point at which the pulse wave has reached its maximum, based on the pulse wave data.

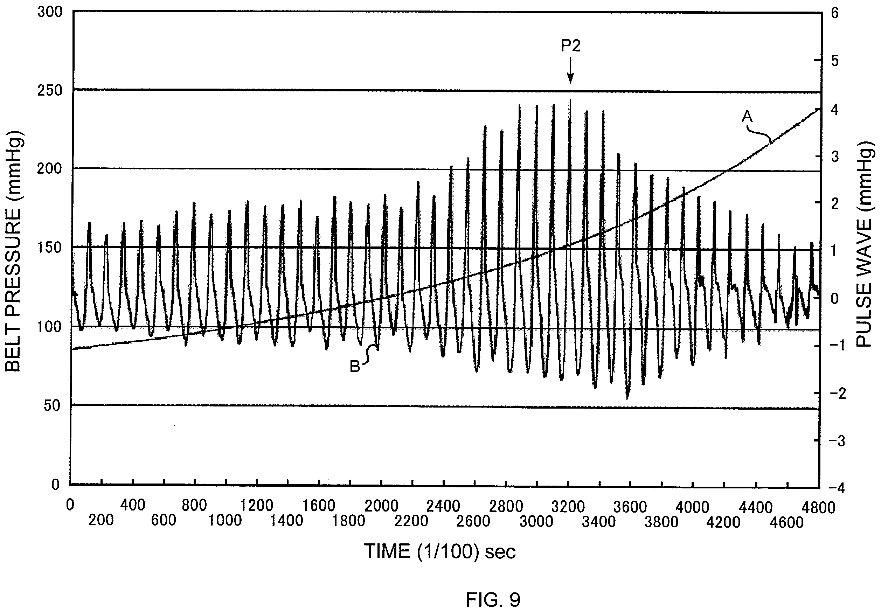

In this modified version, the peak analyzing unit 413 determines the time point at which the amplitude of the pulse wave has reached its maximum, in a manner described below. Fig. 9 shows an example of a measured pulse wave . Depicted by a symbol A in the figure is the pressure within the gas bag 120 (in mmHg) . Depicted by a symbol B is the amplitude of the pulse wave (in mmHg) .

As in this modified version, when the pressure to compress the limb is increased by means of increasing the gas pressure within the gas bag 120, the pulse wave gradually grows as shown in Fig. 9. When the pressure exceeds a certain limit, then the pressure goes into decrease. In Fig. 9, the amplitude of the pulse wave turns from increase to decrease at the time point depicted by a symbol P2 in the figure. The peak analyzing unit 413 in this modified version continuously monitors the amplitude of the pulse wave according to the pulse wave data, as described above. It defines the time point at which the amplitude of the pulse wave turns from increase to decrease, as the time point at which the amplitude of the pulse wave has reached its maximum. It should be noted that the peak analyzing unit 413 may be adapted to define (estimate) the time point at which the pulse wave reaches its maximum using a data indicative of the amplitude of the pulse wave before or before and after the time point depicted by the symbol P2 in the figure, e.g., may be adapted to define (estimate) the time point at which the pulse wave reaches its maximum by differentiating a function about the amplitude of the pulse wave with respect to time to obtain the time point at which the amplitude of the pulse wave reaches its relative maxima, and define (estimate) the time point obtained, as the time point at which the pulse wave reaches its maximum.

At any rate, the peak analyzing unit 413 generates the data indicative of the time point at which the amplitude of the pulse wave has reached its maximum and sends it to the main control unit 412. As described above, the main control unit 412 receives the control data to be used for the preprocessing from the control data generating unit 414. Accordingly, by comparing the variation of the air pressure within the gas bag 120 that can be determined using the control data with the time point at which the amplitude of the pulse wave that can be determined using the data received from the peak analyzing unit 413 has reached its maximum, the main control unit 412 defines the air pressure within the gas bag 120 at the time point at which the amplitude of the pulse wave has reached its maximum, as the maximum pulse wave pressure.

It should be noted that, while the pressure within the gas bag 120 is once increased to a predetermined pressure obviously higher than the maximum pulse wave pressure and then the air pressure within the gas bag 120 is reduced to the normal pressure in the modified version, the pump control mechanism 220 may control the pump to remove the air from the gas bag 120 at the time point at which the peak analyzing unit 413 determines the maximum pulse wave pressure. In this case, the control data generating unit 414 generates a control data to make the pump control mechanism 220 control in such a manner.

### <<Second Embodiment>>

A therapeutic system according to a second embodiment is not significantly different from the therapeutic system according to the first embodiment. A difference between the therapeutic system according to the second embodiment and the therapeutic system according to the first embodiment mainly lies in the structures of the measuring segments 300 and the control segment 400 in the first embodiment.

The measuring segments 300 and the control segment 400 in the first embodiment are not present in the second embodiment.

More specifically, the therapeutic system according to the second embodiment is comprised of a tight fitting device 100 and a body segment 200.

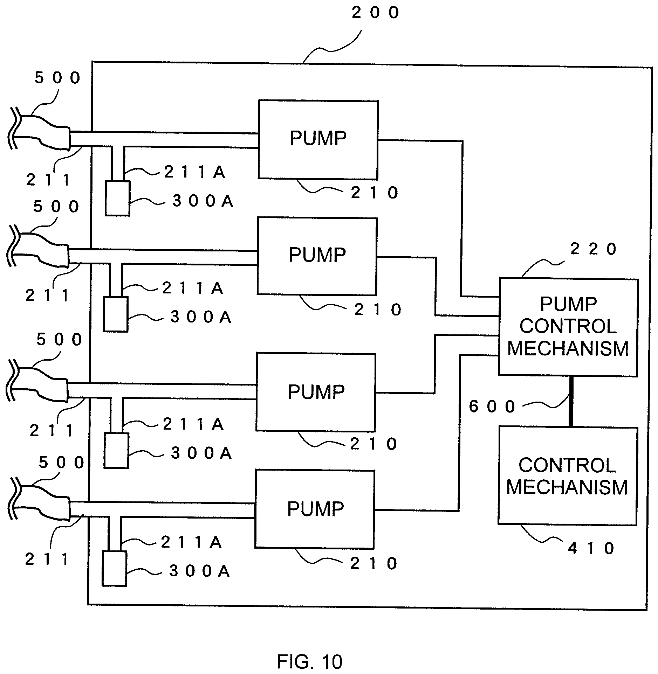

However, the body segment 200 in the therapeutic system according to the second embodiment substantially contains the measuring segments 300 and the control segment 400 in the first embodiment. The internal configuration of the body segment 200 in the second embodiment is shown in Fig. 10.

The body segment 200 in the therapeutic system according to the second embodiment comprises four pumps 210 and a pump control mechanism 220 as in the case of the body segment 200 in the first embodiment. The structure and function of them are identical to those described in the first embodiment. The facts that the pump 210 in the second embodiment comprises a pump connection inlet 211 and is connected through a connecting pipe 500 connected thereto to a gas bag 120 in the tight fitting device 100 are also identical to the first embodiment.

The body segment 200 in the second embodiment contains a control mechanism 410 that has a structure and function identical to the control segment 400 in the first embodiment. The control mechanism 410 contains a computer that is similar to the one contained in the control segment 400 in the first embodiment. This computer has hardware as shown in Fig. 6, as in the case of the first embodiment. In addition, as a CPU 401 executes a program recorded on a ROM 402, functional blocks as shown in Fig. 7 are created within the body segment 200 in the second embodiment, as in the case of the first embodiment. There is no difference in functions of these functional blocks between the second embodiment and the first embodiment.

A branch pipe 211A is connected to the pump connection inlet 211 of each pump 210 contained in the body segment 200 in the second embodiment. The branch pipe 211A is a pipe communicated with the pump connection inlet 211 for branching the pump connection inlet 211. A manometer 300A is attached to the branch pipe 211A at the end thereof to measure the air pressure within the branch pipe 211A. The air pressures across the pump 210, the branch pipe 211A, the pump connection inlet 211, the connecting pipe 500, the connection inlet 121, and the gas bag 120 are of course all equal to each other. Accordingly, the manometer 300A can measure the air pressure within the gas bag 120 by means of measuring the air pressure within the branch pipe 211A.

The aforementioned manometer 300A has a function of successively and continuously measuring, without a break, a predetermined parameter that varies depending on the variation of the amplitude of the pulse wave, as in the case of the measuring segment 300 in the first embodiment. The theory is as follows. The pulse wave is observed on the skin surface as pulsation. In other words, the skin vibrates depending on the periodic increase or decrease of the pulse wave. The vibration is transmitted to the gas bag 120 which is housed in the tight fitting device 100 wrapped around the range to be compressed and which is in contact with the skin. That is, the air pressure within the gas bag 120 changes, although very slightly, due to the pressure from the skin that vibrates depending on the periodic increase or decrease of the pulse wave. The manometer 300A in the second embodiment measures the air pressure within the gas bag 120 by means of measuring the air pressure within the branch pipe 211A, as described above, so that it measures the pressure from the skin that vibrates depending on the pulse wave by measuring the air pressure within the branch pipe 211A. The changing air pressure within the branch pipe 211A represents the parameter that varies depending on the variation of the amplitude of the pulse wave. The data indicative of the changing air pressure within the branch pipe 211A corresponds to the pulse wave data. The four manometers 300A generate such pulse wave data and send them to the control mechanism 410 through a cable which is not shown.

How the pulse wave data is used by the control mechanism 410 is identical to how the pulse wave data is used by the control segment 400 in the first embodiment.

## Claims

1. A therapeutic device that forms a therapeutic system by means of being combined with a tight fitting device (100) including a belt (110) having the length that is enough to be wrapped around a predetermined range of muscles of one of the limbs; fastening means (130) for fastening said belt with said belt being wrapped around said predetermined range of muscles; and a gas bag (120) provided in or on said belt, said gas bag being adapted to apply a predetermined compression pressure to said predetermined range of muscles by means of compressing said predetermined range of muscles when said gas bag is filled with gas while said belt that has been wrapped around said predetermined range of muscles is fastened by said fastening means;
said therapeutic device comprising:
pressure setting means (220) which is capable of setting a gas pressure within said gas bag to a predetermined pressure;
control means (400) for controlling said pressure setting means in order to change said compression pressure; and
pulse wave measuring means (300) for measuring a predetermined parameter that varies according to the variation of amplitude of arterial pulse wave that varies depending on said compression pressure, at a position near said predetermined range of muscles or a position closer to the distal end of the limb than there, to generate a pulse wave data associated with the parameter;
the therapeutic device **characterised in that**:
said control means being adapted to
make said pressure setting means execute two processing steps, i.e., preprocessing and normal processing;
control said pressure setting means during said preprocessing so that said pressure setting means changes the gas pressure within said gas bag in such a manner that said pressure setting means once raises the pressure within said gas bag (120) until it exceeds a pressure that is expected to be higher than the maximum pulse wave pressure and thereafter reduces the pressure within said gas bag, and determine a maximum pulse wave pressure that is a gas pressure within said gas bag at the time point at which the amplitude of the pulse wave has reached its maximum, by means of continuously receiving said pulse wave data from said pulse wave measuring means (300) while the pressure within said gas bag (120) is decreasing during said preprocessing, and determining a maximum pulse wave pressure at the time point at which the pulse wave has reached its maximum, from at least one preceding pulse wave data, when said pulse wave data indicates that the amplitude of said pulse wave becomes smaller than before; and
control said pressure setting means during said normal processing so that said pressure setting means sets the gas pressure within said gas bag to said maximum pulse wave pressure.

2. A therapeutic device that forms a therapeutic system by means of being combined with a tight fitting device including a belt having the length that is enough to be wrapped around a predetermined range of muscles of one of the limbs; fastening means for fastening said belt with said belt being wrapped around said predetermined range of muscles; and a gas bag provided in or on said belt, said gas bag being adapted to apply a predetermined compression pressure to said predetermined range of muscles by means of compressing said predetermined range of muscles when said gas bag is filled with gas while said belt that has been wrapped around said predetermined range of muscles is fastened by said fastening means;
said therapeutic device comprising:
pressure setting means which is capable of setting a gas pressure within said gas bag to a predetermined pressure;
control means for controlling said pressure setting means in order to change said compression pressure; and
pulse wave measuring means for measuring a predetermined parameter that varies according to the variation of amplitude of arterial pulse wave that varies depending on said compression pressure, at a position near said predetermined range of muscles or a position closer to the distal end of the limb than there, to generate a pulse wave data associated with the parameter;
the therapeutic device **characterised in that**:
said control means being adapted to
make said pressure setting means execute two processing steps, i.e., preprocessing and normal processing;
control said pressure setting means during said preprocessing so that said pressure setting means changes the gas pressure within said gas bag in such a manner that said pressure setting means raises a pressure within said gas bag (120) from a pressure that is expected to be lower than the maximum pulse wave pressure, continuously receive said pulse wave data from said pulse wave measuring means (300) while the pressure within said gas bag is increasing, and determine a maximum pulse wave pressure at the time point at which the pulse wave has reached its maximum, from at least one preceding pulse wave data, when said pulse wave data indicates that the amplitude of said pulse wave becomes smaller than before; and
control said pressure setting means during said normal processing so that said pressure setting means sets the gas pressure within said gas bag to said maximum pulse wave pressure.

3. The therapeutic device as claimed in Claim 1, wherein said control means (400) is adapted to continuously receive said pulse wave data from said pulse wave measuring means (300) while the pressure within said gas bag (120) is decreasing during said preprocessing, and adapted to determine an immediately preceding gas pressure within said gas bag as the maximum pulse wave pressure when said pulse wave data indicates that the amplitude of said pulse wave becomes smaller than before.

4. The therapeutic device as claimed in Claim 2, wherein said control means (400) is adapted to control, during said preprocessing, said pressure setting means (220) in such a manner that said pressure setting means (300) raises a pressure within said gas bag from a modified pressure that is expected to be lower than the maximum pulse wave pressure, adapted to continuously receive said pulse wave data from said pulse wave measuring means while the pressure within said gas bag (120) is increasing, and adapted to determine an immediately preceding gas pressure within said gas bag as the maximum pulse wave pressure when said pulse wave data indicates that the amplitude of said pulse wave becomes smaller than before.

5. The therapeutic device as claimed in any one of Claims 1 to 4, wherein said pulse wave measuring means (300) is capable of measuring the gas pressure within said gas bag (120) as said parameter.

6. The therapeutic device of any of the preceding claims, wherein the control means (400) is adapted to differentiate a function about the amplitude of the pulse wave with respect to time to obtain the time point at which the amplitude of the pulse wave reaches its relative maxima, and define the time point obtained, as the time point at which the pulse wave reaches its maximum.

7. A therapeutic system comprising the therapeutic device according to any of the preceding claims and further comprising:
a tight fitting device (100) including a belt (110) having the length that is enough to be wrapped around a predetermined range of muscles of one of the limbs; fastening means (130) for fastening said belt with said belt being wrapped around said predetermined range of muscles; and a gas bag (120) provided in or on said belt, said gas bag being adapted to apply a predetermined compression pressure to said predetermined range of muscles by means of compressing said predetermined range of muscles when said gas bag is filled with gas while said belt that has been wrapped around said predetermined range of muscles is fastened by said fastening means.

8. The therapeutic system as claimed in Claim 7, wherein said therapeutic system comprises a plurality of said tight fitting devices (100A, 100B),
said pulse wave measuring means (300) being equal in number to said tight fitting devices and associated with their respective one of said tight fitting devices, said pulse wave measuring means being adapted to measure said parameter that varies depending on the variation of the amplitude of the pulse wave, at a position near the predetermined range of muscles around which the associated tight fitting device is wrapped, or a position closer to the distal end of the limb than there, and to generate a pulse wave data associated with the limb,
said pressure setting means (220) being equal in number to said tight fitting devices and associated with their respective one of said tight fitting devices,
said control means (400) being adapted to determine, during said preprocessing, the maximum pulse wave pressure for each of the limb, andadaptedto control, during saidnormal processing, each of said pressure setting means associated with said tight fitting device that compresses one of the limbs, so that each of said pressure setting means sets the gas pressure within each gas bag included in the tight fitting device associated with the pressure setting means to said maximum pulse wave pressure.

## Patentansprüche

1. Eine therapeutische Vorrichtung, die ein therapeutisches System bildet, indem sie mit einer enganliegenden Vorrichtung (100) kombiniert wird, die Folgendes umfasst: einen Gurt (110) mit einer Länge, die ausreicht, um einen vorbestimmten Bereich von Muskeln von einem der Gliedmaßen gewickelt zu werden; ein Befestigungsmittel (130) zum Befestigen des Gurts, wobei der Gurt um einen vorbestimmten Muskelbereich gewickelt wird; und einen Gasbeutel (120), der in oder auf dem Gurt bereitgestellt wird, wobei der Gasbeutel dazu angepasst ist, einen vorbestimmten Kompressionsdruck auf den vorbestimmten Muskelbereich auszuüben, indem der vorbestimmte Muskelbereich komprimiert wird, wenn der Gasbeutel mit Gas gefüllt wird, während der Gurt, der um den vorbestimmten Muskelbereich gewickelt worden ist, durch das Befestigungsmittel befestigt ist;
wobei die therapeutische Vorrichtung Folgendes beinhaltet:
ein Druckeinstellmittel (220), das in der Lage ist, einen Gasdruck im Innern des Gasbeutels auf einen vorbestimmten Druck einzustellen;
ein Steuermittel (400) zum Steuern des Druckeinstellmittels, um den Kompressionsdruck zu verändern; und
ein Pulswellenmessmittel (300) zum Messen eines vorbestimmten Parameters, der gemäß der Variation der Amplitude der arteriellen Pulswelle variiert, die in Abhängigkeit von dem Kompressionsdruck variiert, an einer Position in der Nähe des vorbestimmten Muskelbereichs oder einer Position, die näher an dem distalen Gliedmaßenende liegt als dort, um mit dem Parameter assoziierte Pulswellendaten zu erzeugen;
wobei die therapeutische Vorrichtung **dadurch gekennzeichnet ist, dass**:
das Steuermittel dazu angepasst ist,
das Druckeinstellmittel dazu zu veranlassen, zwei Verarbeitungsschritte durchzuführen, d. h. Vorverarbeitung und normale Verarbeitung;
das Druckeinstellmittel während der Vorverarbeitung so zu steuern, dass das Druckeinstellmittel den Gasdruck im Innern des Gasbeutels solcherart verändert, dass das Druckeinstellmittel den Druck im Innern des Gasbeutels (120) einmal erhöht, bis er einen Druck übersteigt, der voraussichtlich höher sein wird als der maximale Pulswellendruck, und den Druck im Innern des Gasbeutels anschließend verringert, und einen maximalen Pulswellendruck zu bestimmen, der ein Gasdruck im Innern des Gasbeutels zu dem Zeitpunkt ist, zu dem die Amplitude der Pulswelle ihr Maximum erreicht hat, indem die Pulswellendaten fortlaufend von dem Pulswellenmessmittel (300) empfangen werden, während der Druck im Innern des Gasbeutels (120) während der Vorverarbeitung abnimmt, und einen maximalen Pulswellendruck zu dem Zeitpunkt, zu dem die Pulswelle ihr Maximum erreicht hat, von mindestens einem der vorhergehenden Pulswellendaten zu bestimmen, wenn die Pulswellendaten anzeigen, dass die Amplitude der Pulswelle kleiner wird als zuvor; und
das Druckeinstellmittel während der normalen Verarbeitung so zu steuern, dass das Druckeinstellmittel den Gasdruck im Innern des Gasbeutels auf den maximalen Pulswellendruck einstellt.

2. Eine therapeutische Vorrichtung, die ein therapeutisches System bildet, indem sie mit einer enganliegenden Vorrichtung kombiniert wird, die Folgendes umfasst: einen Gurt mit einer Länge, die ausreicht, um einen vorbestimmten Bereich von Muskeln von einem der Gliedmaßen gewickelt zu werden; Befestigungsmittel zum Befestigen des Gurts, wobei der Gurt um einen vorbestimmten Muskelbereich gewickelt wird; und ein Gasbeutel, der in oder auf dem Gurt bereitgestellt wird, wobei der Gasbeutel dazu angepasst ist, einen vorbestimmten Kompressionsdruck auf den vorbestimmten Muskelbereich auszuüben, indem der vorbestimmte Muskelbereich komprimiert wird, wenn der Gasbeutel mit Gas gefüllt ist, während der Gurt, der um den vorbestimmten Muskelbereich gewickelt worden ist, durch das Befestigungsmittel befestigt ist;
wobei die therapeutische Vorrichtung Folgendes beinhaltet:
ein Druckeinstellmittel, das in der Lage ist, einen Gasdruck im Innern des Gasbeutels auf einen vorbestimmten Druck einzustellen;
ein Steuermittel zum Steuern des Druckeinstellmittels, um den Kompressionsdruck zu verändern; und
ein Pulswellenmessmittel zum Messen eines vorbestimmten Parameters, der gemäß der Variation der Amplitude der arteriellen Pulswelle variiert, die in Abhängigkeit von dem Kompressionsdruck variiert, an einer Position in der Nähe des vorbestimmten Muskelbereichs oder einer Position, die näher an dem distalen Gliedmaßenende liegt als dort, um mit dem Parameter assoziierte Pulswellendaten zu erzeugen;
wobei die therapeutische Vorrichtung **dadurch gekennzeichnet ist, dass**:
das Steuermittel dazu angepasst ist,
das Druckeinstellmittel dazu zu veranlassen, zwei Verarbeitungsschritte durchzuführen, d. h. Vorverarbeitung und normale Verarbeitung;
das Druckeinstellmittel während der Vorverarbeitung so zu steuern, dass das Druckeinstellmittel den Gasdruck im Innern des Gasbeutels solcherart verändert, dass das Druckeinstellmittel einen Druck im Innern des Gasbeutels (120) von einem Druck, der voraussichtlich niedriger sein wird als der maximale Pulswellendruck, erhöht, fortlaufend die Pulswellendaten von dem Pulswellenmessmittel (300) zu empfangen, während der Druck im Innern des Gasbeutels zunimmt, und einen maximalen Pulswellendruck zu dem Zeitpunkt, an dem die Pulswelle ihr Maximum erreicht hat, von mindestens einem der vorhergehenden Pulswellendaten zu bestimmen, wenn die Pulswellendaten anzeigen, dass die Amplitude der Pulswelle kleiner wird als zuvor; und
das Druckeinstellmittel während der normalen Verarbeitung so zu steuern, dass das Druckeinstellmittel den Gasdruck im Innern des Gasbeutels auf den maximalen Pulswellendruck einstellt.

3. Die therapeutische Vorrichtung gemäß Anspruch 1, wobei das Steuermittel (400) dazu angepasst ist, fortlaufend die Pulswellendaten von dem Pulswellenmessmittel (300) zu empfangen, während der Druck im Innern des Gasbeutels (120) während der Vorverarbeitung abnimmt, und dazu angepasst ist, einen unmittelbar vorhergehenden Gasdruck im Innern des Gasbeutels als den maximalen Pulswellendruck zu bestimmen, wenn die Pulswellendaten anzeigen, dass die Amplitude der Pulswelle kleiner wird als zuvor.

4. Die therapeutische Vorrichtung gemäß Anspruch 2, wobei das Steuermittel (400) dazu angepasst ist, während der Vorverarbeitung das Druckeinstellmittel (220) solcherart zu steuern, dass das Druckeinstellmittel (300) einen Druck im Innern des Gasbeutels von einem modifizierten Druck, der voraussichtlich niedriger sein wird als der maximale Pulswellendruck, erhöht dazu angepasst ist, fortlaufend die Pulswellendaten von dem Pulswellenmessmittel zu empfangen, während der Druck im Innern des Gasbeutels (120) zunimmt, und dazu angepasst ist, einen unmittelbar vorhergehenden Gasdruck im Innern des Gasbeutels als den maximalen Pulswellendruck zu bestimmen, wenn die Pulswellendaten anzeigen, dass die Amplitude der Pulswelle kleiner wird als zuvor.

5. Die therapeutische Vorrichtung gemäß einem der Ansprüche 1 bis 4, wobei das Pulswellenmessmittel (300) in der Lage ist, den Gasdruck im Innern des Gasbeutels (120) als den Parameter zu messen.

6. Die therapeutische Vorrichtung gemäß einem der vorhergehenden Ansprüche, wobei das Steuermittel (400) dazu angepasst ist, eine Funktion über die Amplitude der Pulswelle in Bezug auf die Zeit zu differenzieren, um den Zeitpunkt zu erhalten, an dem die Amplitude der Pulswelle ihre relativen Maxima erreicht, und den erhaltenen Zeitpunkt als den Zeitpunkt zu definieren, zu dem die Pulswelle ihr Maximum erreicht.

7. Ein therapeutisches System, das die therapeutische Vorrichtung gemäß einem der vorhergehenden Ansprüche beinhaltet und ferner Folgendes beinhaltet:
eine enganliegende Vorrichtung (100), die Folgendes umfasst: einen Gurt (110) mit einer Länge, die dazu ausreicht, um einen vorbestimmten Bereich von Muskeln von einem der Gliedmaßen gewickelt zu werden; Befestigungsmittel (130) zum Befestigen des Gurts, wobei der Gurt um den vorbestimmten Muskelbereich gewickelt wird; und einen Gasbeutel (120), der in oder auf dem Gurt bereitgestellt wird, wobei der Gasbeutel dazu angepasst ist, einen vorbestimmten Kompressionsdruck auf den vorbestimmten Muskelbereich auszuüben, indem der vorbestimmte Muskelbereich komprimiert wird, wenn der Gasbeutel mit Gas gefüllt wird, während der Gurt, der um den vorbestimmten Muskelbereich gewickelt worden ist, durch das Befestigungsmittel befestigt ist.

8. Das therapeutische System gemäß Anspruch 7, wobei das therapeutische System eine Vielzahl von enganliegenden Vorrichtungen (100A, 100B) beinhaltet,
wobei das Pulswellenmessmittel (300) in seiner Anzahl gleich der Anzahl von enganliegenden Vorrichtungen ist und mit der jeweiligen der enganliegenden Vorrichtungen assoziiert ist, wobei das Pulswellenmessmittel dazu angepasst ist, den Parameter, der je nach der Variation der Amplitude der Pulswelle variiert, an einer Position in der Nähe des vorbestimmten Muskelbereichs, um den die assoziierte enganliegende Vorrichtung gewickelt wird, oder einer Position, die näher an dem distalen Gliedmaßenende liegt als dort, zu messen und mit der Gliedmaße assoziierte Pulswellendaten zu erzeugen,
wobei das Druckeinstellmittel (220) in seiner Anzahl gleich der Anzahl von enganliegenden Vorrichtungen ist und mit der jeweiligen der enganliegenden Vorrichtungen assoziiert ist,
wobei das Steuermittel (400) dazu angepasst ist, während der Vorverarbeitung den maximalen Pulswellendruck für jede der Gliedmaßen zu bestimmen, und dazu angepasst ist, während der normalen Verarbeitung jedes der Druckeinstellmittel, die mit der enganliegenden Vorrichtung assoziiert sind, die eine der Gliedmaßen komprimiert, zu steuern, so dass jedes der Druckeinstellmittel den Gasdruck im Innern jedes Gasbeutels, der in der enganliegenden Vorrichtung eingeschlossen ist, die mit dem Druckeinstellmittel assoziiert ist, auf den maximalen Pulswellendruck einstellt.

## Revendications

1. Dispositif thérapeutique qui forme un système thérapeutique en étant combiné à un dispositif à ajustement étroit (100) comprenant une bride (110) d'une longueur qui est suffisante pour être enroulée autour d'une gamme prédéterminée de muscles de l'un des membres ; un moyen de fixation (130) pour amarrer ladite bride alors que ladite bride est enroulée autour de ladite gamme prédéterminée de muscles ; et une poche de gaz (120) fournie dans ou sur ladite bride, ladite poche de gaz étant conçue pour appliquer une pression de compression prédéterminée sur ladite gamme prédéterminée de muscles par compression de la gamme prédéterminée de muscles quand ladite poche de gaz est remplie de gaz alors que ladite bride qui a été enroulée autour de ladite gamme prédéterminée de muscles est amarrée par ledit moyen de fixation ;
ledit dispositif thérapeutique comprenant :
un moyen de réglage de la pression (220) permettant de régler une pression de gaz dans ladite poche de gaz à une pression prédéterminée ;
un moyen de contrôle (400) permettant de contrôler ledit moyen de réglage de la pression pour modifier ladite pression de compression ; et
un moyen de mesure de l'onde de pouls (300) permettant de mesurer un paramètre prédéterminé qui varie en fonction de la variation de l'amplitude de l'onde de pouls artérielle qui varie en fonction de ladite pression de compression, situé à une position proche de ladite gamme prédéterminée de muscles ou en un point plus proche de l'extrémité distale du membre que cette position, pour générer des données concernant l'onde de pouls qui sont associées au paramètre ;
le dispositif thérapeutique étant **caractérisé en ce que** :
ledit moyen de contrôle est conçu pour
conduire ledit moyen de réglage de la pression à réaliser deux étapes de traitement, c.-à-d. un prétraitement et un traitement normal ;
contrôler ledit moyen de réglage de la pression pendant ledit prétraitement de sorte que ledit moyen de réglage de la pression modifie la pression de gaz dans ladite poche de gaz de manière à ce que ledit moyen de réglage de la pression produise immédiatement une élévation de la pression dans ladite poche de gaz (120) jusqu'à ce qu'elle dépasse une pression qui est théoriquement plus élevée que la pression au maximum de l'onde de pouls puis une diminution de la pression dans ladite poche de gaz, établit une pression au maximum de l'onde de pouls qui correspond à une pression de gaz dans ladite poche de gaz au jalon temporel auquel l'amplitude de l'onde de pouls a atteint son maximum sur la base des données concernant l'onde de pouls reçues en continu à partir dudit moyen de mesure de l'onde de pouls (300) alors que la pression dans ladite poche de gaz (120) diminue pendant ledit prétraitement, et établit une pression au maximum de l'onde de pouls qui correspond à une pression de gaz dans ladite poche de gaz au jalon temporel auquel l'amplitude de l'onde de pouls a atteint son maximum sur la base d'au moins une donnée précédente concernant l'onde de pouls quand ladite donnée concernant l'onde de pouls indique que l'amplitude de ladite onde de pouls devient plus basse que précédemment ; et
contrôler ledit moyen de réglage de la pression pendant ledit traitement normal de sorte que ledit moyen de réglage de la pression règle la pression de gaz dans ladite poche de gaz à ladite pression au maximum de l'onde de pouls.

2. Dispositif thérapeutique qui forme un système thérapeutique en étant combiné à un dispositif à ajustement étroit comprenant une bride d'une longueur qui est suffisante pour être enroulée autour d'une gamme prédéterminée de muscles de l'un des membres ; un moyen de fixation pour amarrer ladite bride alors que ladite bride est enroulée autour de ladite gamme prédéterminée de muscles ; et une poche de gaz fournie dans ou sur ladite bride, ladite poche de gaz étant conçue pour appliquer une pression de compression prédéterminée sur ladite gamme prédéterminée de muscles par compression de la gamme prédéterminée de muscles quand ladite poche de gaz est remplie de gaz alors que ladite bride qui a été enroulée autour de ladite gamme prédéterminée de muscles est amarrée par ledit moyen de fixation ;
ledit dispositif thérapeutique comprenant :
un moyen de réglage de la pression permettant de régler une pression de gaz dans ladite poche de gaz à une pression prédéterminée ;
un moyen de contrôle permettant de contrôler ledit moyen de réglage de la pression pour modifier ladite pression de compression ; et
un moyen de mesure de l'onde de pouls permettant de mesurer un paramètre prédéterminé qui varie en fonction de la variation de l'amplitude de l'onde de pouls artérielle qui varie en fonction de ladite pression de compression, situé à une position proche de ladite gamme prédéterminée de muscles ou en un point plus proche de l'extrémité distale du membre que cette position, pour générer des données concernant l'onde de pouls qui sont associées au paramètre ;
le dispositif thérapeutique étant **caractérisé en ce que** :
ledit moyen de contrôle est conçu pour
conduire ledit moyen de réglage de la pression à réaliser deux étapes de traitement, c.-à-d. un prétraitement et un traitement normal ;
contrôler ledit moyen de réglage de la pression pendant ledit prétraitement de sorte que ledit moyen de réglage de la pression modifie la pression de gaz dans ladite poche de gaz de manière à ce que ledit moyen de réglage de la pression produise une élévation de la pression dans ladite poche de gaz (120) à partir d'une pression qui est théoriquement plus basse que la pression au maximum de l'onde de pouls, reçoit en continu lesdites données concernant l'onde de pouls à partir dudit moyen de mesure de l'onde de pouls (300) alors que la pression dans ladite poche de gaz augmente et établit une pression au maximum de l'onde de pouls qui correspond au jalon temporel auquel l'amplitude de l'onde de pouls a atteint son maximum sur la base d'au moins une donnée précédente concernant l'onde de pouls quand ladite donnée concernant l'onde de pouls indique que l'amplitude de ladite onde de pouls devient plus basse que précédemment ; et
contrôler ledit moyen de réglage de la pression pendant ledit traitement normal de sorte que ledit moyen de réglage de la pression règle la pression de gaz dans ladite poche de gaz à ladite pression au maximum de l'onde de pouls.

3. Dispositif thérapeutique selon la revendication 1, où ledit moyen de contrôle (400) est conçu pour recevoir en continu lesdites données concernant l'onde de pouls à partir dudit moyen de mesure de ladite onde de pouls (300) alors que la pression dans ladite poche de gaz (120) diminue pendant ledit prétraitement et pour définir une pression de gaz immédiatement précédente dans ladite poche de gaz en tant que pression au maximum de l'onde de pouls quand ladite donnée concernant l'onde de pouls indique que l'amplitude de ladite onde de pouls devient plus basse que précédemment.

4. Dispositif thérapeutique selon la revendication 2, où ledit moyen de contrôle (400) est conçu pour contrôler, pendant ledit prétraitement, ledit moyen de réglage de la pression (220) de manière à ce que ledit moyen de réglage de la pression (300) produise une élévation de la pression dans ladite poche de gaz à partir d'une pression qui est théoriquement plus basse que la pression au maximum de l'onde de pouls, pour recevoir en continu lesdites données concernant l'onde de pouls à partir dudit moyen de mesure de l'onde de pouls alors que la pression dans ladite poche de gaz (120) augmente et pour définir une pression de gaz immédiatement précédente dans ladite poche de gaz en tant que pression au maximum de l'onde de pouls quand ladite donnée concernant l'onde de pouls indique que l'amplitude de ladite onde de pouls devient plus basse que précédemment.

5. Dispositif thérapeutique selon l'une quelconque des revendications 1 à 4, ledit moyen de mesure de l'onde de pouls (300) permettant de mesurer la pression de gaz dans ladite poche de gaz (120) en tant que ledit paramètre.

6. Dispositif thérapeutique de l'une quelconque des revendications précédentes, où le moyen de contrôle (400) est conçu pour différencier une fonction concernant l'amplitude de l'onde de pouls en fonction du temps afin d'obtenir le jalon temporel auquel l'amplitude de l'onde de pouls atteint ses maxima relatifs et de définir le jalon temporel obtenu comme correspondant au jalon temporel auquel l'onde de pouls atteint son maximum.

7. Système thérapeutique comprenant le dispositif thérapeutique selon l'une quelconque des revendications précédentes et comprenant en outre :
un dispositif à ajustement étroit (100) comprenant une bride (110) d'une longueur qui est suffisante pour être enroulée autour d'une gamme prédéterminée de muscles de l'un des membres ; un moyen de fixation (130) pour amarrer ladite bride alors que ladite bride est enroulée autour de ladite gamme prédéterminée de muscles ; et une poche de gaz (120) fournie dans ou sur ladite bride, ladite poche de gaz étant conçue pour appliquer une pression de compression prédéterminée sur ladite gamme prédéterminée de muscles par compression de la gamme prédéterminée de muscles quand ladite poche de gaz est remplie de gaz alors que ladite bride qui a été enroulée autour de ladite gamme prédéterminée de muscles est amarrée par ledit moyen de fixation.

8. Système thérapeutique selon la revendication 7, où ledit système thérapeutique comprend une pluralité desdits dispositifs étroitement ajustés (100A, 100B),
lesdits moyens de mesure de l'onde de pouls (300) étant égaux en nombre auxdits dispositifs étroitement ajustés et associés à leur un desdits dispositifs étroitement ajustés respectifs, lesdits moyens de mesure de ladite onde de pouls étant conçus pour mesurer ledit paramètre qui varie en fonction de la variation de l'amplitude de l'onde de pouls à une position proche de ladite gamme prédéterminée de muscles autour de laquelle le dispositif à ajustement étroit est enroulé ou en un point plus proche de l'extrémité distale du membre que cette position et pour générer une donnée concernant l'onde de pouls associée au membre,
lesdits moyens de réglage de la pression (220) étant égaux en nombre auxdits dispositifs étroitement ajustés et associés à leur un desdits dispositifs étroitement ajustés respectifs,
ledit moyen de contrôle (400) étant conçu pour établir, pendant ledit prétraitement, la pression au maximum de l'onde de pression pour chacun des membres et pour contrôler, pendant ledit traitement normal, chacun desdits moyens de réglage de la pression associés au dit dispositif à ajustement étroit qui comprime l'un des membres de sorte que chacun desdits moyens de réglage de la pression règle la pression du gaz à ladite pression au maximum de l'onde de pouls dans chacune des poches de gaz que comprend le dispositif à ajustement étroit associé au moyen de réglage de la pression.
